# EUROPEAN PATENT APPLICATION

(11) **EP 1 416 032 A1**
(43) Date of publication of application: **06.05.2004**
(21) Application number: 02756030.9
(22) Date of filing: 10.07.2002
(51) Int. Cl.: C09K 21/06, A62C 2/00, A62D 1/02

(54) **INTUMESCENT CARBON-FORMING ANTIPYREN, METHOD OF PRODUCTION AND USE THEREOF**

(30) Priority: 12.07.2001 RU 2001119199
(71) Applicant: Krivosheyev, Sergey Leonidovich, St. Petersburg 193315 (RU)
(72) Inventor: SKIBIDA, Irina Petrovna, Moscow, 117071 (RU); ASEEVA, Poza Mikhailovna, Moscow, 117571 (RU); SAKHAROV, Pavel Andreevich, Moscow, 121096 (RU); SAKHAROV, Andrey Mikhailovich, Moscow, 117279 (RU)
(74) Representative: Viering, Jentschura & Partner
(86) International application number: PCT/RU2002/000330
(87) International publication number: WO 2003/006576

(57) **Abstract**

The invention relates to fire-retardants, i.e. substances protecting organic materials against inflammation and burning, in particular, to intumescent coke-forming fire-retardants. Said fire-retardants are expandable as affected by heat and flame, thereby increasing the volume thereof and forming a solid foamed mass, which exhibits heat-protection properties with respect to underlying material. The single-component systems of intumescent coke-forming fire-retardants based on salts of polyhydroxycarboxylic acids, which are produced by oxidation of carbohydrates are also disclosed.

## Description

### Technical field

The invention relates to fire-retardants being substances and mixtures intended for protection of organic materials against inflammation and burning, particularly, to intumescent coke-forming fire-retardants.

### Description of a Prior Art

Intumescent ("expanding") fire-retardants are substances and mixtures, which are expandable as affected by heat and fire, thereby increasing the volume thereof and forming foamed solid bulk, which exhibits heat-shielding properties. Said foamed bulk acts as a heat protector for an underlying layer of a material.

Different types of intumescent fire-retardants of inorganic and organic nature are known, such as, for example, intumescent inorganic fire-retardants based on sodium silicate, intumescent expanding phylosilicates, expanding natural mineral substances, such as vermiculite.

Another known type of intumescent fire-retardants comprises materials, which form cellular carbon framework having heat-shielding properties after expanding or foaming. Such fire-retardants include, for example, expanding carbon, single-component low-molecular compounds, particularly, 4,4-dinitrosulfanilide, aryl sulfonamide derivatives.

Multi-component intumescent fire-retardant systems forming foamed carbonated materials as affected by heat are also known. Typically, such systems include the following components:
1) A source of coke-forming foam framework;
2) A source of gasification and foaming;
3) A catalyst (activator) for reactions of dehydration, cross-linking of macromolecules and coke formation of an organic component;
4) A binder.

Certain binders (urea- and melamine- formaldehyde, polyurethane resins) can act concurrently as a coke-forming agent and an inflating foaming agent.

Conventional foaming fire-retardant systems are mono- and di- ammonium phosphate, polyammonium phosphate, melamine derivatives of polyphosphoric acids in a combination with polyols, for example, pentaerythritol (bis-hydroxy-methyl).

One of the most important components of intumescent fire-retardant system is a coke-forming component. Low-molecular and high-molecular polyols, sugars, starch, dextrin are typically used as said component. Phosphoric acid salts and derivatives are typically used as coke-forming activator and nitrogenous compounds (such as melamine, urea, dicyanamide etc.) are used as foaming agents.

U.S. Patent 3,956,236 (E. F. Evans et al.) discloses fire-retardant synergistic compositions comprising metals and ammonium salts of low-molecular hydroxycarboxylic acids.

U.S. Patent 4,061,810 (Pritam Singh Minhas et al.) discloses fire-retardant systems for decreasing combustibility of polyamide and polyester coatings being a physical mixture of metal oxides and hydroxides (such as oxides and hydroxides of Sn, Al, Zn and Sb) with low-molecular hydroxycarboxylic acids (such as citric, tartaric, gallic acids).

EP 0625561 B1 (Giovando, Gualtiero) discloses fire-retardants for various organic materials comprising low-molecular polyhydroxycarboxylic acids (citric, tartaric, mucic, glycolic, glyoxylic, gluconic, glucaric, saccharic and lactic acids) in a combination with ammonium salts capable of forming complex (coordination) compounds with hydroxycarboxylic and other acids.

The closest prior art for the claimed intumescent coke-forming fire-retardant are fire-retardants, which disclosed in U.S. Patent 3,943,100 (M.B. Berenbaum et al.). Said fire-retardants are based on certain low-molecular organic hydroxycarboxylic acids (tartaric, citric and gallic) and ammonium, lithium and magnesium salts thereof.

As a rule, intumescent fire-retardants are used for fire-protective treatment of a flammable substrate as a film coating a surface of said substrate, or as a solution for impregnating porous flammable substrates (such as paper of textile), or as an additive (filling agent) introduced into polymer materials for decreasing combustibility thereof (see, for example, Patent RU 2026310 and Patent RU 2026311). Such treatment reduces a probability of inflaming the material treated thereby.

Various methods are used for seat of fire extinguishing, such as treatment of the seat of fire with foam, with gases, which do not sustain combustion, or with aerosols (fine-dispersed solid or liquid compositions or substances capable of extinguish a fire) sprayed in air medium. Thus, Patent RU 2123366 discloses a method for fire extinguishing with sprayed liquid.

Though many different fire-retardants of organic and inorganic nature are known, there is a need in diversification thereof and in discovering fire-retardants having high efficacy and low toxicity, which can be produced from inexpensive raw materials.

### Summary of the Invention

The problem to be solved is to provide an inexpensive, effective and environmentally safe fire-retardant.

Said problem is solved by using salts of polyhydroxycarboxylic acids, which can be obtained by oxidation of carbohydrates conducted with substitution a metal or ammonium cation for a hydrogen ion of a carboxyl group of obtained polyhydroxycarboxylic acids, as intumescent coke-forming fire-retardant.

The inventors found out that the salts of polyhydroxycarboxylic acids, which are obtained by oxidation of low-molecular and high-molecular carbohydrates to polyhydroxycarboxylic acids and substitution a metal or ammonium cation for a hydrogen ion of a carboxyl group of said polyhydroxycarboxylic acids resulting in generation of corresponding salts of said polyhydroxycarboxylic acids, are capable to expand (to foam) forming extensional foamed coke as affected by high temperature and naked flame. Said foamed extensional coke is capable to provide fire protection for a flammable substrate. On the base of this discovering the inventors drew a conclusion that said salts may be used as intumescent fire-retardants.

The main advantages of intumescent coke-forming fire-retardants based on salts of polyhydroxycarboxylic acids (hereinafter also designated as "POA") according to the present invention are as follows.

The claimed fire-retardants:
- are compounds being inexpensive and environmentally safe;
- do not evolve high-toxic volatile products when burning and degrading;
- exhibit low fume evolution during smoldering and flame combustion;
- can be obtained in a soluble form starting from inexpensive available plant raw materials, waste of production thereof and recycling of corresponding materials;
- are high-performance fire-retardants.

According to the invention, salts of polyhydroxycarboxylic acids as intumescent coke-forming fire-retardants combine several functions of components of complex fire-retardant systems, particularly, said salts are simultaneously:
- a source for forming a carbided framework;
- a foaming agent;
- catalysts of a basic type for reactions resulting in carbonization; and
- high-molecular salts of POA are able to act as binding agents of carbonizing type.

Experiments, which were carried out by the inventors, showed that salts of polyhydroxycarboxylic acids, which are obtained by different processes of oxidation of different carbohydrates (mono-, di- and polysaccharides) using various cations, are capable of foaming forming solid carbided framework as affected by high temperature and fire. Consequently, said salts may be used as intumescent coke-forming fire-retardants.

On the grounds of the carried out experiments the inventors drew a conclusion that various types of carbohydrates including monosaccharides, disaccharides and polysaccharides may be used as raw materials for producing fire-retardants according to the invention. Preferably, monosaccharides are selected from the group comprising pentoses and hexoses including arabinose, glucose and fructose; disaccharides may be selected from maltose, saccharose, cellobiose and trehalose; polysaccharides may be selected from dextrines, dextranes, starch, components thereof (amylose and amylopectin), hydrolysates thereof as well as cellulose.

Taking into consideration economic factor as well as effectiveness, the most preferable polysaccharides for producing fire-retardants according to the invention are starch and cellulose. Starch is optionally dissolved, or dispersed, or solubilized in water prior to oxidation. Cellulose, being a material insoluble in water, is optionally dispersed and/or solubilized in water prior to its oxidation for producing salts of POA. Particularly, cellulose may be solubilized by adding alkali and alkaline-earth metals hydroxides thereto.

The most convenient and, therefore, the most preferable source for producing intumescent coke-forming fire-retardant of the invention are plant raw materials. Suitable plant raw materials include, particularly, treacle or grain (corn) as well as products derived from it (such as flour, cereals, groats, extrusion starch) or plant waste. Wood including wood products and wood waste (for example, sawdust or shavings) also may be used for production fire-retardant of the invention.

It should be understood that intumescent coke-forming fire-retardant of the invention can be obtained by various known processes which are used for producing salts of polyhydroxycarboxylic acids starting from carbohydrates. Particularly, the processes disclosed in U.S. Patent 5,484,914, EP 0755944, in *P. Parovuri, A. Hamunen, P. Forsell, K*. *Autio, K*. *Poutanen*. *Starch 47* (1995) *Nº 1, p. 19-23*, or *B. Casu, U. Gennaro, S. V. Meille, M. Morrone, A. Naggi, M. S. Occhipinti, G. Torri.* // *Int. J. Biol. Macromol., 1984, Vol. 6, April, p. 89-92* or other processes of obtaining salts of polyhydroxycarboxylic acids from carbohydrates are suitable for producing the fire-retardant of the invention.

Thus, the salts of polyhydroxycarboxylic acids applicable as intumescent coke-forming fire-retardants may be produced, for example, by oxidizing carbohydrates with oxidants, particularly, with oxygen, oxygen-containing gas, hydroperoxide, organic peroxides, hypochlorites, periodates, permanganates, iodides, bromites. A cation of the generated salts of polyhydroxycarboxylic acids may be a cation contained in the oxidant and/or a cation, which is added to the reaction medium during or after oxidation. The added cation may be introduced to a reaction medium in a form base or in a form of inorganic or organic salt.

Experiments showed that degree of expansion (foaming) of the salts of POA and yield of coke (carbon residue) arising from the effect of fire and high temperature depend on chemical nature of the polyhydroxycarboxylic acids as well as on nature of a cation. Cations of metals of Group I of the periodic table as well as ammonium cation or cations of metals of Groups II-VIII of the periodic table or combinations thereof may be used as a cation forming the fire-retardants according to the present invention. Sodium and potassium salts of polyhydroxycarboxylic acids produced according to the present invention are the most preferable salts for use as intumescent coke-forming fire-retardants.

Combination of different cations in a molecular structure of POA can provide synergistic increasing in efficiency of the claimed fire-retardants, improvement of barrier properties of heat-insulating foamed coke.

Salts of POA of the invention may be used as intumescent coke-forming fire-retardant for protection of organic materials in various forms. Thus, the fire-retardant may be used in a form of water solutions for surface and volume fire-protective treatment of combustible substrate as well as in a form of dry powder introduced into polymer compositions.

Thus, the surface fire-protecting treatment is performed by applying aqueous solution of the fire-retardant of the invention on a surface of a combustible substrate and drying it to form a fire-retardant film (i.e. a film of the salts of POA) on said surface.

Treatment of porous combustible substrates (such as textiles or paper) in volume is implemented by impregnating said substrate with aqueous solution of said fire-retardant and subsequent drying it to humidity corresponding to the humidity of environment.

Treatment in volume of combustible substrates being polymer compositions may be performed by introducing said fire-retardant in a form of dry powder as a supplement to said polymer composition.

Experiments carried out by the inventors showed that aqueous solutions of the salts of POA sprayed in air medium may be used as effective fire-fighting means. So, another aspect of the invention is a method for extinguishing a seat of fire comprising treatment of said seat of fire with a fine-dispersed gas-liquid blend (aerosol), which is formed as a result of spraying fire extinguishing liquid being an aqueous solution of the fire-retardant of the invention in air medium.

### Detailed Description of the Invention

The following Examples illustrate the present invention.

### Example 1

A 100 ml thermostatically controlled glass reactor provided with a rabble is charged with:
- 50 ml of water,
- 20 g of sucrose,
- 0.39 g of CuSO₄·5H₂O as a catalyst of an oxidation reaction and
- 2.5 g of NaOH as co-catalyst and salt-forming agent for generating salts from polyhydroxycarboxylic acids, which are formed as a result of oxidizing.

The reactor is attached to a gas-meter filled with oxygen, is blown with pure oxygen in order to drive out air, and is sealed. The mixture is heated to 75°C and stirred. Oxidation is carried out during 6 hours and is stopped after completion of consumption of oxygen caused by depletion of alkali owing to its neutralizing with oxyacids formed as a result of the reaction (see U.S. Patent 5,484,914, January 16, 1996).

Product is dried in air and thin films of the salts of polyhydroxycarboxylic acids are obtained thereby. The obtained dry material in a form of films is heated in air at 250 ± 0.5°C during 10 minutes. Foaming capability is computed as ratio of volume of formed foamed coke to the volume of initial dry material (2 g). Then loss of mass of the material is determined.

Sodium salt of POA obtained as a result of oxidation of sucrose melts and "boils up" forming reticular foam as affected by heat. A coefficient of expansion (of foaming) of the material is K = 10.8 and residual weight is 10.6 % by weight based upon the initial weight of the sample of the material.

### Example 2

Oxidation of maltodextrin is performed according to a procedure disclosed in EP 0755944 A2 (publication date January 29, 1997, Bulletin 1997/05), similarly to the process described in Example 1. 50 ml of water, 25 g of maltodextrin having molecular weight 200÷300 and 1.25 g of NaOH are placed into the reactor. Oxidation is carried out during 3 hours at a temperature of 75°C.

Weight of an obtained sample (measured after drying in air) is 0.25g. Coefficient of expansion of sodium salt of POA determined according to Example 1 is K = 25.6. Residual weight is 61.9%. Sample of non-oxidized maltodextrin under the same conditions does not boil up and loses 16.5% of its weight.

### Example 3

Oxidation of potato starch is performed as described in Example 1. 50 ml of water, 20 g of potato starch and 0.06 g of CuSO₄·5H₂O are placed into the reactor. A solution is stirred during 5 minutes, and then 2.5 g of NaOH is added. An oxidizing cell is blown with oxygen; the solution is heated to 75°C and stirred. Oxidation is carried out during 6 hours. After oxidation, the solution is dried in air and thin films of salts of POA are obtained.

A sample having weight 40-50 g is heated in air at dynamic conditions (at a heating rate of 10°C/min.) in derivatograph Q-1500 D (Hungary). Sodium salt of POA degrades with foaming in the temperature range of from 173°C to 298°C. Weight of carbon residue (coke) at a temperature of 530°C is 41% of the weight of initial sample.

Coefficient of expansion of a dry sample (0.25g) determined according to Example 1 (under isothermal conditions) is K = 32. Residual weight is 64.2 %.

### Example 4

Oxidation of potato starch is performed as described in Example 3. 12.5 g of AlCl₃·6H₂O is added to an aqueous solution of sodium salts of POA. Films of the salts of POA are obtained after dissolving aluminium chloride by drying the obtained solution in air.

Under dynamic heating carried out according to Example 3 a sample degrades with foaming in the temperature range of from 194°C to 490°C. Weight of carbon residue (coke) at a temperature of 530°C is 33.3% by weight based upon the initial weight of the sample of the material.

Under isothermal conditions coefficient of expansion of the dry sample (0.25g) is K = 29 and residual weight is 61 %.

### Example 5

Oxidation of potato starch is performed as described in Example 3. 2.5 g of H₃BO₃ is added to an aqueous solution of sodium salts of POA. Films of the salts of POA are obtained after dissolving boric acid by drying the obtained solution in air.

Under dynamic heating a sample degrades and foams in the temperature range of from 240°C to 350°C. Weight of carbon residue (coke) at a temperature of 530°C is 44.7% of the weight of the initial sample. The coke is stable in air up to 670°C.

Under isothermal conditions at 250°C coefficient of expansion of the dry sample (0.25g) is K = 2.6. Weight of carbon residue is 77.9 %.

### Example 6

Oxidation of potato starch is performed as described in Example 3. 25 g of KCr(SO₄)₂·12H₂O is added to an aqueous solution of sodium salts of POA. Films of the salts of POA are obtained after dissolving KCr(SO₄)₂·12H₂O by drying the obtained solution in air.

Under dynamic heating performed according to Example 3 a sample degrades and foams in the temperature range of from 210°C to 340°C. Maximal rate of degradation is observed at Tₘₐₓ = 278°C. Weight of carbon residue at a temperature of 530°C is 54.9%.

Under isothermal conditions of Example 1 coefficient of expansion of the sample is K = 2.4. As affected by flame of a gas-jet during 2 minutes the sample does not inflame, but carbonizes and foams. After removal of the flame, smoldering is not observed.

### Example 7

Oxidation of potato starch is performed as described in Example 3. 12.5 g of MnSO₄·5H₂O is added to an aqueous solution of sodium salts of POA. Films of the salts of POA are obtained after dissolving manganese sulfate by drying the obtained solution in air.

Under dynamic heating performed according to Example 3 a sample degrades with foaming at a temperature of from 217°C to 369°C. Weight of carbon residue at a temperature of 530°C is 45.7%.

Under isothermal conditions of Example 1 coefficient of expansion of the sample is K = 1.4. Residual weight is 80.4 %

As affected by flame of a gas-jet during 2 minutes the sample does not inflame, but carbonizes. Smoldering is not observed.

### Example 8

Oxidation of potato starch is performed as described in Example 3. 2.5 g of ZnCl₂ is added to an aqueous solution of sodium salts of POA. Films of the salts of POA are obtained after dissolving zinc chloride by drying the obtained solution in air.

Under conditions of Example 1 coefficient of expansion of the sample weighing 3.05 g is K = 14. Residual weight is 64.4 %

As affected by flame of a gas-jet during 2 minutes the sample foams and carbonizes. After removing the flame, smoldering of the sample is observed.

### Example 9

Oxidation of potato starch is performed as described in Example 1. 2.5 g of SnCl₂·2H₂O is added to an aqueous solution of sodium salts of POA. Films of the salts of POA are obtained after dissolving tin chloride by drying the obtained solution in air.

Under conditions of Example 1 coefficient of expansion of the sample (2.5 g) is K = 3.0. Residual weight is 67.3 %

As affected by flame of a gas-jet the sample foams and carbonizes. After removing the flame, smoldering of the sample is not observed.

### Example 10

Oxidation of potato starch is performed as described in Example 3. 2.5 g of Cu(HCOO)₂ is added to an aqueous solution of sodium salts of POA. Films of the salts of POA are obtained after dissolving cuprum formate by drying the obtained solution in air.

Under conditions of Example 1 coefficient of expansion of the sample is K = 27.1. Residual weight is 66.4 %

As affected by flame of a gas-jet the sample does not inflame; quick foaming and carbonizing is observed. Smoldering is not observed.

### Example 11

Oxidation of rice is performed as described in Example 3. 50 ml of water, 12 g of rice and 0.12 g of CuSO₄·5H₂O are placed into the reactor. After dissolving copper sulfate, 2 g of granulated NaOH is added into an oxidizing cell. The oxidizing cell is blown with oxygen, the cell is thermostatically controlled at 70°C and solution is stirred. Oxidation is carried out during 8 hours. After oxidation, the solution is dried in air and thin films of salts of POA are obtained.

Coefficient of expansion determined according to Example 1 is K = 29.8. Residual weight is 61.4 %.

Under dynamic heating performed according to Example 3 foaming is observed at a temperature above 188°C. Weight of carbon residue at a temperature of 530°C is 41.3%.

### Example 12

Oxidation of potato starch is performed in a thermostatically controlled 1-liter glass reactor provided with a rabble according to a procedure disclosed in P. Parovuri, A. Hamunen, P. Forsell, K. Autio, K. Poutanen. Starch 47 (1995) Nº 1, p. 19-23. 100 g of dry starch is dispersed in water forming 42% dispersion. 0.5 g of FeSO₄·7H₂O is dissolved in water before dispersing the starch. pH of a reaction medium is adjusted at pH=10 by adding 2M water solution of NaOH. Temperature is adjusted at 40°C. Solution of 30% hydrogen peroxide is introduced into the reaction medium by drops, in order to provide constant concentration of H₂O₂ (about 2%) in the reaction medium. Oxidation is carried out during 15 hours. After completion of the reaction the obtained salts of the oxidized starch is filtered in Buchner funnel and dried in air.

Under dynamic heating performed according to Example 3 degradation and foaming is observed in the temperature range of from 180°C to 450°C. Weight of carbon residue at a temperature of 530°C is 27.3%.

Coefficient of expansion of the dry sample (0.25g) is K = 17. Residual weight under isothermal conditions is 42 %.

### Example 13

Oxidizing of amylose (molecular weight 150000, Serva) with sodium metaperiodate is performed according to a procedure disclosed in B. Casu, U. Gennaro, S. V. Meille, M. Morrone, A. Naggi, M. S. Occhipinti, G. Torri. // Int. J. Biol. Macromol., 1984, Vol. 6, April, p. 89-92. Salts of POA obtained thereby are dried in air.

Under dynamic heating performed according to Example 3 degradation and foaming of the salts of POA is observed in the temperature range of from 175°C to 440°C. Weight of carbon residue at a temperature of 530°C is 29.5%.

Coefficient of expansion of a dry sample (0.25g) is K = 19. Residual weight under isothermal conditions is 39 %.

### Example 14

Films of salts of POA are obtained as described in Example 3. 50 ml of water, 20 g of potato starch and 0.06 g of CuSO₄·5H₂O are placed into the reactor. A solution is stirred during 5 minutes and then 2.5 g of NaOH is added. An oxidizing cell is blown with oxygen; the solution is heated to 75°C and stirred. Oxidation is carried out during 6 hours. After oxidation, the solution is dried in air and thin films of salts of POA are obtained.

Fume evolution and toxicity of the products of combustion of the sample is determined according to par. 4.18 and 4.20 of GOST 12.2.044-89 (State Standard) under the most dangerous conditions of thermal-oxidative degradation (smoldering), namely, under heating of a surface of the sample to a temperature of 400 ± 5°C and heat-flux density qₑ = 18 ± 1 kW/m². According to the results of determination of fume evolution, the sample is classified as a material of Group D2. The fume evolution of the sample is Dₘ^{max} = 71.5 m²/kg. According to the results of determination of toxicity (toxicity index HCL₅₀ = 57.7 g/m³) the sample is related to Group T2 as a moderately toxic material.

### Example 15

Films of salts of POA are obtained as described in Example 11.

Fume evolution index determined as described in Example 14 is Dₘ^{max} = 75.9 m²/kg (Group D2). Toxicity index: HCL₅₀ = 203.2 g/m³. The sample is related to Group T1 as low-hazard material. Lower heat of complete combustion is 14.482 kJ/g.

### Example 16

Potato starch is oxidized according to the procedure described in Example 3. 50 ml of water, 20 g of potato starch and 0.1 g of CuSO₄·5H₂O are placed into the reactor. A solution is stirred during 5 minutes, and then 3.5 g of NaOH is added. An oxidizing cell is blown with oxygen; the solution is heated to 75°C and stirred. Oxidation is carried out during 10 hours. After oxidation, the solution is dried in air and thin films of salts of POA are obtained.

Under dynamic heating carried out according to Example 3 a sample degrades with foaming in the temperature range of from 196°C to 318°C. Weight of carbon residue (coke) at a temperature of 530°C is 39%.

Under isothermal conditions (heating at 250°C during 10 minutes) coefficient of expansion (0.25g) is K = 17.6 and residual weight is 60 %.

Products of smoldering combustion of the sample examined according to Example 14 have fume evolution index of Dₘ^{max} = 42.5 m²/kg. The sample is related to Group D1 as a material having low fume evolution. Toxicity index HCL₅₀ = 53.4 g/m³. The sample is related to Group T2 as a moderately toxic material. Lower heat of complete combustion is 14.757 kJ/g.

### Example 17

Oxidation of rice is performed similarly to Example 11. 50 ml of water, 12.5 g of rice and 0.1 g of CuSO₄·5H₂O are placed into the reactor. After dissolving cuprum sulfate 0.8 g of granulated NaOH is added into an oxidizing cell. The oxidizing cell is blown with oxygen, the cell is thermostatically controlled at 70°C and solution is stirred. Oxidation is carried out during 4 hours. After drying the solution in air thin films of salts of POA are obtained.

Under dynamic heating performed according to Example 3 the sample degrades and foams at a temperature of from 212°C to 330°C. At a temperature of 560°C weight of carbon residue is 20.5%.

As affected by of flame of a gas-jet the sample foams, carbonizes, coke glows, but after removal of the flame smoldering terminates.

Mixture of dry PA-6,6 (Polyacrylamide-6,6) with 5% of dry powder of POA salts demonstrates that under heating in air with heating rate of 10°C/min. melting of the polymer becomes slower. Temperature of beginning the melting of the mixture (235°C) is higher than the corresponding temperature for pure PA-6,6 (226°C). Temperature of beginning degradation of the mixture of PA-6,6 with 5% of salts of POA (330°C) is lower than the corresponding temperature for pure PA-6,6 (351°C).

Weight of carbon residue of the mixture at a temperature of 530°C is 43.7%, whereas pure PA-6,6 loses 80% of its weight being heated to 500°C.

Effectiveness of the salts of POA as coke-forming fire-retardants is illustrated by the following examples.

### Example 18

Samples of filter and writing paper, as well as viscose textile of 0.1 mm thickness are treated with aqueous solution of sodium salts of POA produced by oxidation of carbohydrates according to Example 16. Weight increment determined after drying the treated samples at room temperature is 10-15 g/m². Oxygen index (Ol) is determined according to par. 4.14 of GOST 12.1.044-89. After treatment with the salts of POA, the oxygen index increased from 19.5 % to 24.7 % for filter paper, from 20.2 % to 35.2 % for writing paper and from 19.7 % to 25.3 % for viscose textile.

Increasing of content of the salts of POA (calculated based on weight increment of a dried sample) in viscose textile to 30-60 g/m² results in increasing of Ol to 28-30 %.

### Example 19

Waste products of timber industry being pine sawdust are mixed with 40% aqueous solution of a salt of POA in a ratio of 90:10 calculated based on weight of dry materials. The salt of POA is obtained as described in Example 11 and is used as binding agent. After drying and pressing at pressure P = 25 kg/m² and temperature T = 80-100°C samples of wood chipboard having thickness of 3-4 mm are obtained.

Oxygen index of the obtained samples is Ol = 40-42%. Index of flame spreading determined according to par. 4.19 of GOST 12.1.044-89 is I_{fs} = 12-15.

### Example 20

Surface of samples of pine are treated with 40% aqueous solution of sodium salt of POA obtained according to Example 15 and are dried at room temperature during 24 hours. Efficiency of fire-protective effect of a coating based on the salt of POA is determined according to GOST 16363 and GOST 30402-96 (equivalent to ISO 5657). At consumption of the salt of POA equal to 80-100 g/m² the coating provides protection corresponding to Group G1 of fire-protective efficiency according to GOST 16363 and Group V1 of flammability according to GOST 30402-96. Critical external radiation heat flow for inflammation is within a range of from 23 to 29.7 kW/m². Critical external heat flow for inflammation of samples of untreated pine is 12.5 kW/m².

### Example 21

Influence of adding of the salts of POA produced according to Example 3 on effectiveness of fire extinguishing with water aerosol is estimated by decrease of average time required for extinguishing of burning of cotton textile. An experimental seat of file is extinguished with water aerosol during 15.5 seconds. Supplement of the salts of POA in amount 50-150 g/l to the water forming aerosol permits to decrease the time required for fire extinguishing to 10.0-6.2 seconds correspondingly.

## Claims

1. An intumescent coke-forming fire-retardant comprising one or more of salts of polyhydroxycarboxylic acids, **characterized in that** said salts of polyhydroxycarboxylic acids are obtained by oxidation of carbohydrates to polyhydroxycarboxylic acids and substitution a metal or ammonium cation for a hydrogen ion of a carboxyl group of said polyhydroxycarboxylic acids.

2. The fire-retardant according to claim 1, **characterized in that** said salts of polyhydroxycarboxylic acids are simultaneously: a source for forming carbided framework of foam, a foaming agent, a catalysts of reactions resulting in carbonization and a binding agent.

3. The fire-retardant according to any of claims 1 and 2, **characterized in that** said carbohydrates are selected from the group comprising monosaccharides, disaccharides and polysaccharides.

4. The fire-retardant according to claim 3, **characterized in that** said monosaccharides are selected from the group comprising native pentoses and hexoses including arabinose, glucose and fructose; said disaccharides are selected from maltose, saccharose, cellobiose and trehalose; said polysaccharides are selected from the group comprising dextrines, dextranes, starch, starch hydrolysates and cellulose.

5. The fire-retardant according to claim 4, **characterized in that** said starch comprises amylose and/or amylopectin.

6. The fire-retardant according to any of claims 1 or 2, **characterized in that** said carbohydrates are starch being dissolved, or dispersed, or solubilized in water.

7. The fire-retardant according to any of claims 1 or 2, **characterized in that** said carbohydrates are cellulose being dispersed and/or solubilized in water.

8. The fire-retardant according to claim 7, **characterized in that** cellulose is solubilized in water by adding hydroxide of alkali or alkaline-earth metal.

9. The fire-retardant according to any of the preceding claims, **characterized in that** said carbohydrates are derived from plant raw material.

10. The fire-retardant according to claim 9, **characterized in that** said plant raw material is grain, or products derived from grain including flour, cereals and/or extrusion starch, or grain waste.

11. The fire-retardant according to claim 9, **characterized in that** said plant raw material is treacle.

12. The fire-retardant according to claim 9, **characterized in that** said plant raw material is wood, or wood production, or wood waste products including sawdust and/or shavings.

13. The fire-retardant according to any of the preceding claims, **characterized in that** the carbohydrates are oxidized with an oxidant selected from the group comprising: oxygen, oxygen-containing gas, hydroperoxide, organic peroxides, hypochlorites, permanganates, periodates, iodides, bromites.

14. The fire-retardant according to any of the preceding claims, **characterized in that** said salts of polyhydroxycarboxylic acids are salts of metals of Group I of the periodic table, preferably sodium salts and potassium salts.

15. The fire-retardant according to any of claims 1-13, **characterized in that** said salts of polyhydroxycarboxylic acids are ammonium salts, or salts of metals of Groups II-VIII of the periodic table, or combination thereof.

16. The fire-retardant according to any of the preceding claims, **characterized in that** said fire-retardant comprises only the salts of polyhydroxycarboxylic acids or aqueous solution thereof.

17. The fire-retardant according to any of claims 1-15, **characterized in that** said fire-retardant is a dry powder of the salts of polyhydroxycarboxylic acids.

18. The fire-retardant according to any of claims 1-15, **characterized in that** said fire-retardant is an aqueous solution of the salts of polyhydroxycarboxylic acids.

19. The fire-retardant according to claim 18, **characterized in that** it is intended for fire extinguishing by spraying in a form of fine dispersion.

20. A method of producing intumescent coke-forming fire-retardant of organic nature, said method comprising oxidation of carbohydrates to polyhydroxycarboxylic acids and substitution a metal or ammonium cation for a hydrogen ion of a carboxyl group in the obtained polyhydroxycarboxylic acids resulting in generation of salts of said polyhydroxycarboxylic acids.

21. The method according to claim 20, **characterized in that** said carbohydrates are selected from the group comprising monosaccharides, disaccharides and polysaccharides.

22. The method according to claim 21, **characterized in that** said monosaccharides are selected from the group comprising native pentoses and hexoses including arabinose, glucose and fructose; said disaccharides are selected from maltose, saccharose, cellobiose and trehalose; said polysaccharides are selected from the group comprising dextrines, dextranes, starch, starch hydrolysates and cellulose.

23. The method according to claim 20, **characterized in that** said carbohydrates are starch, and the method additionally comprises a step of dissolving, or dispersing, or solubilizing the starch in water preceding the step of oxidation.

24. The method according to claim 20, **characterized in that** said carbohydrates are cellulose, and the method additionally comprises a step of dispersing and/or solubilizing the cellulose in water preceding the step of oxidation.

25. The method according to claim 24, **characterized in that** the solubilization of cellulose is carried out by adding hydroxide of alkali or alkaline-earth metal thereto.

26. The method according to any of claims 20-25, **characterized in that** said carbohydrates are derived from plant raw material.

27. The method according to claim 26, **characterized in that** said plant raw material is grain, or products derived from grain including flour, cereals and/or extrusion starch, or grain waste.

28. The method according to claim 26, **characterized in that** said plant raw material is treacle.

29. The method according to claim 26, **characterized in that** said plant raw material is wood, or wood production, or wood waste products including sawdust and/or shavings.

30. The method according to any of claims 20-29, **characterized in that** the carbohydrates are oxidized with oxidant selected from the group comprising: oxygen, oxygen-containing gas, hydroperoxide, organic peroxides, hypochlorites, permanganates, periodates, iodides, bromites.

31. The method according to any of claims 20-30, **characterized in that** said cation is a cation of metal of Group I of the periodic table, preferably sodium or potassium cation.

32. The method according to any of claims 20-30, **characterized in that** said cation is a cation of metal of Groups II-VIII of the periodic table, or ammonium cation.

33. The method according to any of claims 20-32, **characterized in that** said cation is added into a reaction medium after completion of the reaction of oxidation.

34. The method according to any of claims 20-32, **characterized in that** said cation presents in a reaction medium during the reaction of oxidation.

35. A method of fire-protective treatment of a combustible substrate with fire-retardant, **characterized in that** said fire-retardant is the fire-retardant as defined in any of claims 1-16.

36. The method according to claim 35, **characterized in that** aqueous solution of said fire-retardant is applied of a surface of said combustible substrate and is dried to form a fire-retardant film on the surface of said combustible substrate.

37. The method according to claim 35, **characterized in that** said combustible substrate is impregnated with aqueous solution of said fire-retardant and is dried to humidity corresponding to the humidity of environment.

38. The method according to claim 34, **characterized in that** said combustible substrate is a polymer composition, and that said fire-retardant is introduced as a supplement in a form of dry powder to said polymer composition.

39. A method for extinguishing a seat of fire comprising treatment of said seat of fire with a fine-dispersed gas-liquid blend, which is formed as a result of spraying fire extinguishing liquid in air medium, **characterized in that** said liquid is an aqueous solution of the fire-retardant as defined in any of claims 1-16, 18 or 19.
